(19) Europäisches Patentamt
European Patent Office
Office européen des brevets

(11) Publication number: **0 447 706 A1**

(12) **EUROPEAN PATENT APPLICATION**

(21) Application number: **90303116.9**

(22) Date of filing: **22.03.90**

(51) Int. Cl.5: **C07J 13/00**, A61K 31/57

(43) Date of publication of application:
**25.09.91 Bulletin 91/39**

(84) Designated Contracting States:
**CH DE FR GB IT LI**

(71) Applicant: **CIPLA LIMITED**
**289, Bellasis Road, Bombay Central**
**Bombay-400008, Maharashtra(IN)**

(72) Inventor: **Hamied, Yusuf Khawja, Windsor Villa, 2nd Floor**
**Westfield Estate, Off Bhulabhai Desai Road**
**Post Office Bombay 400026, Maharashtra(IN)**

(74) Representative: **Pendlebury, Anthony et al**
**PAGE, WHITE & FARRER 54 Doughty Street**
**London WC1N 2LS(GB)**

(54) **A process for the preparation of pharmacologically active synthetic z and e steroisomeric mixture of guggulsterones.**

(57) There is provided a process for the preparation of pharmacologically active synthetic Z and E stereoisomeric mixture of guggulsterones by reacting a mixture of 5,17(20)-trans-pregnadiene-3β, 16β and 3β, 16∝ diols in an aromatic solvent in the presence of a catalyst using a hydrogen acceptor at reflux temperature in an inert atmosphere.

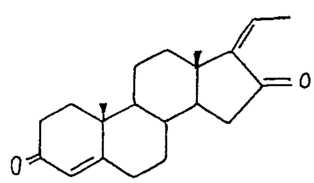

FORMULA I A

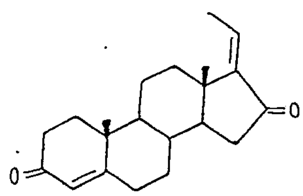

FORMULA I B

FIGURE 1

EP 0 447 706 A1

This invention relates to a process for the preparation of pharmacologically active synthetic z and E stereoisomeric mixture of guggulsterones of the formulae IA and IB of the accompanying drawings, respectively.

Guggul-resin/gum (oleo-resin) is obtained from the tree Commiphora mukul (Hook, ex stocks), family Burseraceae. This resin/gum is known as Guggulu in Sanskrit and Guggul in Hindi. In Indian Patent Specification No.148265 there is described a process for obtaining hypolipaedemic and antiplatelet aggregation fraction from guggul-resin by subjecting the resin to extraction with ethyl acetate and separating out the extract. The ethyl acetate extract is an oily resinous substance and is termed as 'Gugulipid' and its chemical analysis has established the presence of two known ketonic steriods-guggulsterones (Z and E isomers) in the ratio of about 80:20. The medicinal activity of 'Gugulipid' is attributed to these steroids. 'Gugulipid' is also known to contain many other steroids and non-ketonic compounds exerting synergistic action on the biological activity of the ketonic fraction. 'Gugulipid'is also known to contain diterpenoid hydrocarbon, related alcohol and lignans. The chemical activity of 'Gugulipid' is similar to that of the well known drug Clofibrate but 'Gugulipid' is without the side effects associated with Clofibrate and, therefore, preferable and safe. But the tree namely Commiphora mukul (Hook, ex stocks) having right quantities of Z and E stereoisomeric mixture of guggulsterones is not easily available and a large number of such trees has to be examined for the right quantities of Z and E stereoisomeric mixture of guggulsterones. Furthermore, being an oily substance 'Gugulipid' is not easily amenable to compressible dosage forms such as tablets or dragees.

An object of the present invention is to provide a process for the preparation of pharmacologically active synthetic Z and E stereoisomeric mixture of guggulsterones of the formulae IA and IB of the accompanying drawings, respectively.

According to the present invention there is provided a process for the preparation of pharmacologically active synthetic Z and E stereoisomeric mixture of guggulsterones of the formulae IA and IB of the accompanying drawings, respectively, comprising reacting 5, 17(20)-trans-pregnadiene-3$\beta$, 16$\beta$ and 3$\beta$, 16$\alpha$ diols mixture in an aromatic solvent in the presence of a catalyst using a hydrogen acceptor at reflux temperature in an inert atmosphere such as argon or nitrogen atmosphere to obtain the Z and E stereoisomeric mixture of guggulsterones and isolating and purifying the Z and E stereoisomeric mixture of guggulsterones from the reaction mixture in a known manner.

The aromatic solvent is, for example, toluene, xylene, cumene or methyl ethyl ketone.

The catalyst is, for example, aluminium isopropoxide, aluminium isobutoxide, aluminium phenoxide or aluminium tertiary butoxide.

The hydrogen acceptor is, for example, cyclohexanone, cycloheptanone or bemophenone.

One known manner of isolating and purifying the Z and E stereoisomeric mixture of guggulsterones from the reaction mixture consists of cooling the reaction mixture to room temperature, treating the reacting mixture with sulphonic acid, separating the organic layer and washing it with water and drying it over sodium sulphate, treating the residue with active charcoal and recrystallising it from acetone.

The chemical composition of the synthetic Z and E stereoisomeric mixture of guggulsterones of the present invention in the ratio of about 80:20 was established by High Pressure Liquid Chromatography and has been found to be identical to that of natural Z and E stereoisomeric mixture of guggulsterones contained in 'Gugulipid'. The synthetic Z and E stereoisomeric mixture of guggulsterones of the present invention has also been found to possess diverse biological/pharmalogical activities such as hypolipedemic, anti-obesity and hypocholesterolemic activities. Biological testing in rats and mice with both the synthetic and natural Z and E stereoisomeric mixtures of guggulsterones showed no difference between the two. Thus the present invention provides the unique advantage of obtaining the Z and E stereoisomeric mixture of guggulsterones synthetically thereby obviating the disadvantages associated with the obtaining of natural Z and E stereoisomeric mixture of guggulsterones. Furthermore, the synthetic Z and E stereoisomeric mixture of guggulsterones is obtained in solid form and is amenable to compressible solid dosage forms such as tablets or dragees.

The following examples illustrate the present invention but do not limit the scope thereof.

example 1

A solution of 5,17(20) -trans-pregnadiene-3$\beta$, 16$\beta$ and 3$\beta$, 16$\alpha$ diols (50g) in toluene (2L), dry cyclohexanone (200 ml), and aluminium isoproproxide (20g) were refluxed for 2 hours under nitrogen. The reaction mixture was cooled to room temperature and was shaken with 1% sulphonic acid (1L). The toluene layer was separated and washed with water (75ml x 3) and dried over $Na_2SO_4$ and recrystallised from acetone after treatment with active charcoal to give a Z and E stereoisomeric mixture of guggulsterones

about (80:20) as solid, m.p. 180-183 °C.

The starting material was prepared as follows :

To a cooled solution of 16-dehydropregnalone acetate (50g) in tetrahydrofuran (1.5L) at 0 °C was added with stirring a suspension of $LiACl_4$ (90%, 10g) in tetrahydrofuran (75ml) during 50 minutes. The reaction mixture was stirred for 2 hours at 0 °C and extracted with ethyl acetate (75ml) and the ethyl acetate extract was precipitated with $Na_2SO_4$ saturated solution in water (75 ml). The precipitate was removed by filtration and washed with water (50ml x 3) and dried over $Na_2SO_4$ to give 5,16-pregnadiene-3β-20 diol (44g), m.p. 179-181 °C.

A solution of 5,16-pregnadiene-3β-20 diol (44g) in acetic acid (1L) was allowed to stand in acetic anhydride (150 ml) and p-toluene sulphonic acid (2.5g) at 18-26 °C for 72 hours. The reaction mixture was extracted with chloroform (250 ml x 2), the organic layer was separated and washed with 10% $Na_2CO_3$ -(60ml x 3), water (60ml x 3), and brine (60ml x 3) and dried over $Na_2SO_4$ to give a mixture of 5,17(20)-transpregnadiene-3β, 16β and 3β, 16α diacetates (44.5g) as a semi-solid.

A solution of 44.5g of 5,17(20)-trans-pregnadiene-3β,16β and 3β,16α diacetates mixture in 10% mathanolic-KOH (100g in 900 ml methanol) was refluxed for 6 hours in nitrogen atmosphere. After cooling the reaction mixture to room temperature, water (300 ml) was added and methanol was distilled off under reduced pressure. Water (150 ml) was added to the residue and extracted with chloroform (250ml x 2). The organic layer was washed with water (60ml x 3) and brine (60ml x 3) and dried over $NA_2SO_4$ to give 5,17 (20)-transpregnadiene-3β, 16β and 3β,16α diols (40g), m.p. 174-178 °C.

example 2

A solution of 5,17(20)-trans-pregnadiene-3β,16β and 3β, 16α diols (50g) in toluene (2L), dry cycloheptanone (200 ml) and aluminium isobutoxide (20g) were refluxed for 2 hours under nitrogen. The reaction mixture was cooled to room temperature and worked up in the manner described in Example 1 to give a Z and E stereoisomeric mixture of guggulsterones (about 80:20) as solid, m.p. 180-183 °C.

example 3

A solution of 5,17(20)-trans-pregnadiene-3β,16β and 3β,16α diols (50g) in dry toluene (1,8L) dry cyclohexanone (200 ml) and aluminium phenoxide (50g) were refluxed for 6 hours under nitrogen. The reaction mixture was cooled to room temperature and worked up in the manner described in Example 1 to give a Z and E stereoisomeric mixture of guggulsterones (80:20) as solid, m.p. 180-182 °C.

## Claims

1. A process for the preparation of a pharmacologicallly active synthetic Z and E stereoisomeric mixture of guggulsterones of the following formulae IA and IB respectively:

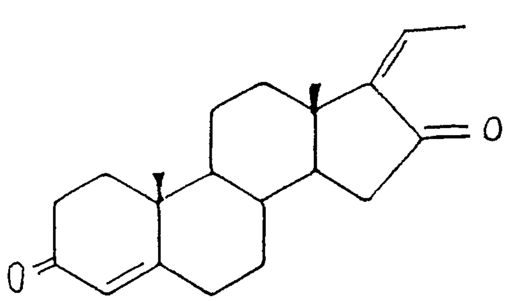

IA

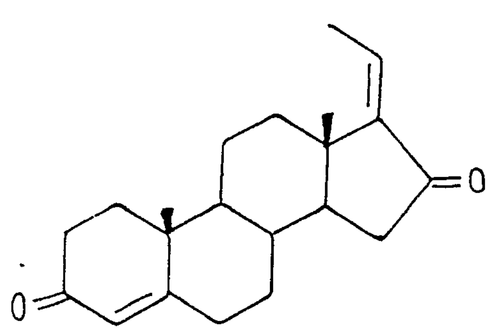

IB

which process comprises reacting a mixture of 5,17(20)-trans-pregnadiene-3β, 16β and 3β, 16α diols in an aromatic solvent in the presence of a catalyst using a hydrogen acceptor at reflux temperature in an inert atmosphere to obtain the Z and E stereoisomeric mixture of guggulsterones and purifying the Z

and E stereoisomeric mixture of guggulsterones from the reaction mixture.

2. A process as claimed in claim 1 wherein the inert atmosphere comprises an argon or nitrogen atmosphere.

3. A process as claimed in claim 1 or claim 2 wherein the aromatic solvent is toluene, xylene, cumene or methyl ethyl ketone.

4. A process as claimed in any one of claims 1 to 3 wherein the catalyst comprises aluminium isopropoxide, aluminium isobutoxide, aluminium phenoxide or aluminium tertiary butoxide.

5. A process as claimed in any one of the preceding claims wherein the hydrogen acceptor comprises cyclohexanone, cycloheptanone or bemophenone.

6. A process as claimed in any one of the proceding claims where the step of purifying comprises cooling the reaction mixture to room temperature, treating the reaction mixture with sulphonic acid, separating an organic layer, washing the organic layer with water and drying the washed organic layer over the sodium sulphate, treating the formed residue with active charcoal and recrystallising from acetone.

7. A pharmaceutical composition comprising a pharmacologically active synthetic Z and E stereoisomeric mixture of guggulsterones obtained from the process of any one of the proceding claims and a pharmaceutically-acceptable carrier or diluent.

8. A process for the preparation of pharmacologically active synthetic Z and E stereoisomeric mixture of guggulsterones of the formulae IA and IB of the accompanying drawings, respectively, comprising reacting 5,17(20)-trans-pregnadiene-3$\beta$, 16$\beta$ and 3$\beta$, 16$\alpha$ diols mixture in an aromatic solvent in the presence of a catalyst using a hydrogen acceptor at reflux temperature in an inert atmosphere such as argon or nitrogen atmosphere to obtain the Z and E stereoisomeric mixture of guggulsterones and isolating and purifying the Z and E stereoisomeric mixture of guggulsterones from the reaction mixture in a known manner.

9. A process for the preparation of pharmacologically active synthetic Z and E stereoisomeric mixture of guggulsterones of the formulae IA and IB of the accompanying drawings, respectively substantially as herein described.

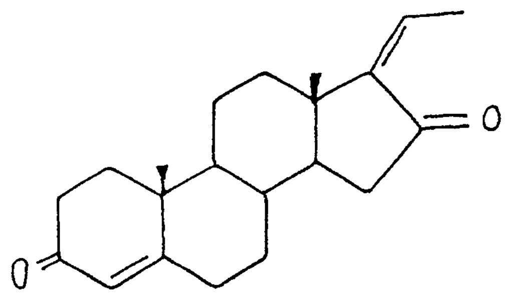

FORMULA I A

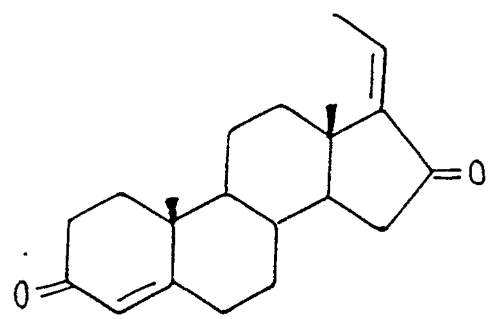

FORMULA I B

FIGURE 1

European Patent Office

# EUROPEAN SEARCH REPORT

Application Number

**EP 90 30 3116**

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | TETRAHEDRON, vol. 38, no. 19, 1982, pages 2949-2954, Pergamon Press, Ltd, Oxford, GB; A.G. BAJAS et al.: "Chemistry of ayurvedic crude drugs-V. Guggulu (resin from Commiphora mukul)-5. Some new steroidal components and, stereochemistry of guggulsterol-1 at C-20 and C-22" * Whole document * --- | 7 | C 07 J 13/00<br>A 61 K 31/57 |
| A | IDEM --- | 1 | |
| X | CHEMICAL ABSTRACTS, vol. 92, no. 15, 14th April 1980, page 34, abstract no. 121687n, Columbus, Ohio, US; L. MESTER et al.: "Inhibition of platelet aggregation by "guggulu" steroids", & PLANTA MED. 1979, 37(4), 367-9 * Abstract * --- | 7 | |
| X | CHEMICAL ABSTRACTS, vol. 101, no. 11, 10th September 1984, page 60, abstract no. 83952q, columbus, Ohio, US; Y.B. TRIPATHI et al.: "Thyroid stimulating action of Z-guggulsterone obtained from Commiphora mukul", & PLANTA MED. 1984, 50(1), 78-80 * Abstract * --- | 7 | **TECHNICAL FIELDS SEARCHED (Int. Cl.5)** |
| X | CHEMICAL ABSTRACTS, vol. 105, no. 9, 1st September 1986, page 91, abstract no. 72860q, Columbus, Ohio, US; M. SRIVASTAVA et al.: "Guggulsterone induced changes in the levels of biogenic monoamines and dopamine beta-hydroxylase activity of rat tissues", & J. BIOSCI. 1986, 10(1), 15-19 * Abstract * --- | 7 | C 07 J 13/00<br>A 61 K 31/00 |
| A | THE JOURNAL OF ORGANIC CHEMISTRY, vol. 29, no. 5, 12th May 1964, pages 1142-1148, American Chemical Society; W.R. BENN et al.: "The synthesis and stereochemistry of isomeric 16-hydroxy-17(20)-pregnenes" * Whole document * --- | 1 | |

-/-

The present search report has been drawn up for all claims

| Place of search | Date of completion of search | Examiner |
|---|---|---|
| The Hague | 19 November 90 | WATCHORN P.W. |

CATEGORY OF CITED DOCUMENTS

- X: particularly relevant if taken alone
- Y: particularly relevant if combined with another document of the same catagory
- A: technological background
- O: non-written disclosure
- P: intermediate document
- T: theory or principle underlying the invention
- E: earlier patent document, but published on, or after the filing date
- D: document cited in the application
- L: document cited for other reasons
- &: member of the same patent family, corresponding document

European Patent Office

# EUROPEAN SEARCH REPORT

Application Number

EP 90 30 3116

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A | TETRAHEDRON, vol. 28, no. 8, April 1972, pages 2341-2352, Pergamon Press, Oxford, GB; V.D. PATIL et al.: "Chemistry of Ayurvedic crude drugs-I. Guggulu (resin from Commiphora mukul)-1: Steroidal constituents" * Page 2342 * | 1 | |
| | | | TECHNICAL FIELDS SEARCHED (Int. Cl.5) |

The present search report has been drawn up for all claims

| Place of search | Date of completion of search | Examiner |
|---|---|---|
| The Hague | 19 November 90 | WATCHORN P.W. |

CATEGORY OF CITED DOCUMENTS

X: particularly relevant if taken alone
Y: particularly relevant if combined with another document of the same catagory
A: technological background
O: non-written disclosure
P: intermediate document
T: theory or principle underlying the invention

E: earlier patent document, but published on, or after the filing date
D: document cited in the application
L: document cited for other reasons

&: member of the same patent family, corresponding document